# EUROPEAN PATENT SPECIFICATION

(11) **EP 3 996 495 B1**
(45) Date of publication and mention of the grant of the patent: **19.06.2024**
(21) Application number: 20737629.4
(22) Date of filing: 01.07.2020
(51) Int. Cl.: A01J 5/013, G01N 33/04, A01J 5/007

(54) **MILK ANALYSIS APPARATUS AND TEMPERATURE ADJUSTMENT THEREIN**
MILCHANALYSEGERÄT UND TEMPERATURREGELUNG DARIN
APPAREIL D'ANALYSE DE LAIT ET RÉGLAGE DE TEMPÉRATURE EN SON SEIN

(30) Priority: 09.07.2019 SE 1950873
(43) Date of publication of application: 18.05.2022
(73) Proprietor: DeLaval Holding AB, 147 21 Tumba (SE)
(72) Inventor: CARLSEN, Thomas Nikolai, 147 21 Tumba (SE); SLAABY, John, 147 21 Tumba (SE)
(74) Representative: DeLaval International AB
(86) International application number: PCT/SE2020/050694
(87) International publication number: WO 2021/006797

(56) References cited:
- WO-A1-2019/132760
- US-A1- 2005 003 522

## Description

### TECHNICAL FIELD

This document discloses a milk analysis apparatus. More particularly, a milk analysis apparatus is presented, associated with a milking equipment, configured for receiving a cassette detachably inserted into the milk analysis apparatus, which cassette comprises a first part for maintaining unused lateral flow sticks and a second part for maintaining used lateral flow sticks. The temperature within the housing of the milk analysis apparatus is measured via a temperature sensor and compared with a temperature threshold limit. Based on the outcome of the comparison, the temperature within the housing may be adjusted.

### BACKGROUND

On an animal farm, it is important to keep the animals healthy in order to enhance milk/ meat production. For example, it is important to inseminate animals at an optimal moment in order to successfully fertilise the cow/ animal. In case the animal is not successfully inseminated, milk production is affected.

Several biomarker measurements may be made on the animal, such as e.g. measuring levels of progesterone, LDH (Lactate Dehydrogenase), BHB (Beta-Hydroxybutyrat) and urea. Thereby important information concerning e.g. heat detection and/ or pregnancy of the individual animal may be made (based on measured progesterone level), as well as mastitis (based on LDH) and ketosis (based on BHB). Also, the energy balance may be estimated (based on urea).

Thereby, a farmer/ operator is provided with important information concerning each individual animal. The analysation of the animals may be made automatically by a milk analysis apparatus, for example as presented in document WO 2018236271. Thereby lateral flow sticks/ milk analysis units/ dry sticks may be easily administrated at the farm by forwarding one lateral flow stick at the time. One lateral flow stick is typically used for each test sample. The cassette may comprise a large amount of lateral flow sticks, yet the cassette with the lateral flow sticks has to be exchanged for a new one when all the lateral flow sticks have been consumed.

A barn wherein animals are maintained typically does not have neither central heating nor air conditioning facilities. Sometimes, the barn is constructed without insulation. Thus, temperature inside the barn may vary over the year depending on environmental temperature, but also depending on the number of animals in the barn in relation to the barn volume.

In summertime, the temperature occasionally may become very high (depending on geographical location of the farm), which may affect the reliability of the lateral flow sticks, as the chemicals used on the lateral flow sticks/ milk analysis units/ dry sticks ages faster in high temperature and may not behave as expected if exposed for extreme temperatures. Measurement devices, sensors, processors and other electronic equipment of the milk analysis apparatus may also be affected. Every animal in the barn radiates body heat, which adds to the overall inside temperature in the barn and thereby also increased temperature at the milk analysis apparatus and the lateral flow sticks.

The opposite problem may also occur in particular in wintertime e.g. in subarctic or tempered climate zones; i.e. that it becomes very cold in the barn, or at least in the location where the milk analysis apparatus is situated. Also, a too low temperature may affect the behaviour and reliability of the lateral flow sticks, as the reaction when the milk sample is added to the lateral flow stick is delayed in low temperature. The result of the test may thereby become too slow or become unreliable.

Another problem at the farm/ barn maintaining animals is that the environment often comprises dust and insects, which may affect the milk analysis apparatus (e.g. the sensible electronics therein) and/ or the lateral flow sticks, if for example generous ventilation holes are drilled into the milk analysis apparatus for increasing ventilation.

It would for these reasons be advantageous to find a manner of controlling/ adjusting the temperature of a milk analysis apparatus at a farm and enable cooling of the milk analysis apparatus when the temperature exceeds a threshold limit, thereby assuring reliable results of biomarker measurements made by the milk analysis apparatus on a lateral flow stick.

Document WO 2019/132760 A1 discloses a cassette comprising dry sticks for measuring biomarkers in milk. The cassette is provided with ventilation apertures for keeping the temperature around the dry sticks above 10 degrees and below 25 degrees.

### SUMMARY

It is therefore an object of this invention to solve at least some of the above problems related to extreme temperatures and deteriorated chemicals on lateral flow sticks to enable reliable biomarker measurements of a milk sample of an animal.

According to a first aspect of the invention, this objective is achieved by a milk analysis apparatus associated with a milking equipment, according to claim 1.

The cassette comprises lateral flow sticks for analysing a milk sample.

By allowing the cassette to be detachably inserted into the milk analysis apparatus, it becomes possible to in a convenient manner exchange the cassette, also for the unexperienced operator.

The purpose of the milk analysis apparatus is to analyse milk extracted from animals at a farm via the milking equipment. The analysis may concern a biomarker of the milk. The analysis may be made by applying a milk sample on a lateral flow stick on the cassette and analyse a colour change of the lateral flow stick.

A biomarker, or biological marker, generally refers to a measurable indicator of some biological state or condition of the animal. The biomarker value measurement may be associated with pregnancy/ reproduction of the animal such as progesterone measurements.

The milk analysis apparatus comprises a receiving section, configured to detachably receive the cassette. Also, the milk analysis apparatus comprises a housing enclosing at least a part of the milk analysis apparatus. The milk analysis apparatus furthermore comprises a thermoelectric element. Additionally, the milk analysis apparatus comprises an interior heat exchange element, arranged adjacent to the thermoelectric element. Furthermore, the milk analysis apparatus comprises a fan, arranged for circulating air inside the housing of the milk analysis apparatus. In further addition, the milk analysis apparatus also comprises a temperature sensor arranged inside the housing of the milk analysis apparatus. The milk analysis apparatus in addition comprises a guided surface of the milk analysis apparatus for guiding circulating air inside the housing via the interior heat exchange element towards an outlet of the receiving section.

The outlet of the receiving section of the milk analysis apparatus may in turn be situated at or close to an aperture/ air guidance means, arranged on a first part of the cassette wherein unused lateral flow sticks are maintained, for directing the circulating air to the unused lateral flow sticks in the cassette.

The milk analysis apparatus also comprises a control unit. The control unit is configured to determine temperature inside the housing via the temperature sensor. Also, the control unit is configured to compare the determined temperature with a temperature threshold limit. The control unit is also configured to adjust current provided to the thermoelectric element, based on the comparison.

The thermoelectric element may comprise e.g. a Peltier element. The thermoelectric element is based on thermoelectric effect by conversion of electric voltage difference to a temperature difference between two sides of the thermoelectric element, a warm side and a cold side. When current is applied to the thermoelectric element, heat is transferred from the warm side to the cold side, creating a temperature difference. By adjusting the current provided to the thermoelectric element, the temperature difference between the two sides of the thermoelectric element may thereby be adjusted. Increased current leads to increased temperature difference and vice versa. The hot side and the cold side of the thermoelectric element could be reversed by reversing the current provided to the thermoelectric element. The control unit could hereby adjust whether heating or cooling of the inside of the housing is desired.

At an atomic scale, an applied temperature gradient causes charge carriers in the material to diffuse from the hot side to the cold side. As the direction of heating and cooling is determined by the polarity of the applied voltage, the thermoelectric element can be used as temperature controller.

Thanks to the guided surface, a closed loop air circuit is created inside the milk analysis apparatus, resulting in a controlled temperature therein. By measuring the inside temperature with the temperature sensor and comparing it with a threshold limit, voltage may be applied to the thermoelectric element for controlling the temperature difference between the warm side and the cold side of the thermoelectric element is adjusted. Also, the speed of the fan may be adjusted for adjusting the air circulation inside the milk analysis apparatus, thereby adjusting the inside temperature. Hereby, a controlled temperature is achieved in the milk analysis apparatus which always remains below the temperature threshold limit, or within a desired temperature interval, leading to reliable biomarker tests at the milk analysis apparatus. The desired temperature interval may be set to about 20-30 degrees.

In a first possible implementation of the milk analysis apparatus according to the first aspect, the milk analysis apparatus may comprise an exterior heat exchange element arranged on the external side of the housing. Also, the milk analysis apparatus may comprise the interior heat exchange element arranged on the internal side of the housing of the milk analysis apparatus, adjacent to the thermoelectric element. The thermoelectric element of the milk analysis apparatus may be arranged on the external side of the housing of the milk analysis apparatus with a first side adjacent to the exterior heat exchange element and a second side adjacent to the housing.

By having an exterior heat exchange element outside the housing of the milk analysis apparatus, and an interior heat exchange element inside the housing of the milk analysis apparatus, which heat exchange elements are situated adjacent to a respective side of the thermoelectric element, an effective heat exchange with the environment is achieved.

In a second possible implementation of the milk analysis apparatus according to the first possible implementation of the first aspect, the milk analysis apparatus may also comprise a thermal conducting plate arranged in an opening of the housing.

The thermal conducting plate may with advantage be made in a material with favourable thermal conducting properties, such as e.g. aluminium, copper or an alloy comprising aluminium and/ or copper.

Aluminium is a metal having excellent thermal conducting properties, while being relatively cheap, lightweight and is easily machined. The low melting temperature of 660 °C allows for easy casting. By arranging the aluminium plate in the opening of the housing, heat absorbed from the interior heat exchange element inside the milk analysis apparatus is transferred to the cold side of the thermoelectric element, or vice versa.

The temperature inside the milk analysis apparatus is thereby controlled while maintaining the closed design of the insulated housing of the milk analysis apparatus.

Further, the second side of the thermoelectric element may be situated adjacent to the exterior side of the thermal conducting plate. The interior heat exchange element may be situated adjacent to the interior side of the thermal conducting plate.

Thanks to the thermal conducting plate and by placing one side of the thermoelectric element adjacent to the exterior side of the thermal conducting plate, it is possible to heat or chill the interior of the housing also when the thermoelectric element is situated outside the housing and thereby adjust the temperature.

In a third possible implementation of the milk analysis apparatus according to the first aspect, or any implementation thereof, the guided surface of the milk analysis apparatus may also comprise a first segment for guiding circulating air inside the housing to pass the heat exchange element towards an outlet of the receiving section of the milk analysis apparatus. The guided surface may also comprise a second segment for guiding circulating air inside the housing from the outlet of the receiving section of the milk analysis apparatus back towards the heat exchange element.

Hereby the guided surface of the milk analysis apparatus is arranged for creating a loop of circulating air passing the heat exchange element and the outlet of the receiving section of the milk analysis apparatus. Via the outlet of the receiving section, the circulating air is directed towards the aperture/ air guidance means of the cassette towards the first part of the cassette wherein unused lateral flow sticks are maintained. Thereby, the temperature of the unused lateral flow sticks in the cassette is efficiently maintained within a predetermined or configurable temperature interval.

According to the invention, the fan is a radial fan, arranged on the interior of the housing, for creating an airflow through the interior heat exchange element, thereby bringing the air to circulate within the housing.

Radial fans have the advantage that they are very energy efficient and can realise high pressure. Radial fans are particularly suitable to displace air in over-pressure situations and ensure a stable laminar air flow.

In a fourth possible implementation of the milk analysis apparatus according to the first aspect, or any implementation thereof, the temperature sensor may be arranged adjacent to the outlet of the receiving section.

By measuring the temperature close to the unused lateral flow sticks in the cassette yet having the temperature sensor in the milk analysis apparatus and not in the cassette, the temperature of the unused lateral flow sticks could be estimated.

In a fifth possible implementation of the milk analysis apparatus according to the first aspect, or any implementation thereof, the thermoelectric element may be arranged on one of the external lateral sides of the milk analysis apparatus.

The thermoelectric element such as Peltier elements are very sensible for liquid. By arranging the thermoelectric element on any lateral side of the milk analysis apparatus, moisture damage of the thermoelectric element is avoided. Hereby, reliability of the temperature control is increased.

In a sixth possible implementation of the milk analysis apparatus according to the first aspect, or any implementation thereof, the housing enclosing the milk analysis apparatus comprises an insulated housing.

By insulating the housing for example by a layer of e.g. Styrofoam or similar material; or two parallel walls with an air gap in between, it becomes easier to maintain a consistent temperature within the housing.

In an seventh possible implementation of the milk analysis apparatus according to the first aspect, or any implementation thereof, the guided surface is part of an internal wall of the milk analysis apparatus, possibly made by injection moulding or similar production process. Thereby a swift production of the milk analysis apparatus is assured.

In a eighth possible implementation of the milk analysis apparatus according to the first aspect, or any implementation thereof, the control unit may be configured to compare the determined temperature with an upper temperature threshold limit set to about 30 degrees and a lower temperature threshold limit set to about 20 degrees. The control unit may also be configured to increase current provided to the thermoelectric element for increasing the temperature when the temperature inside the housing is determined to be lower than the lower temperature threshold limit; or decrease current provided to the thermoelectric element for decreasing the temperature when the temperature inside the housing is determined to be higher than the upper temperature threshold limit.

Thanks to the temperature control, the temperature around the lateral flow sticks in the cassette may be kept at the temperature between 20-30 degrees, leading to more reliable results of the biometric measurements.

By the described aspects, biomarker values of milk samples of animals on the farm may be measured in an automatised manner, yet reliable thanks to avoidance of excessive temperatures, requiring a minimum of efforts of an operator.

Other advantages and additional novel features will become apparent from the subsequent detailed description.

### FIGURES

Embodiments of the invention will now be described in further detail with reference to the accompanying figures, in which:
- **Figure 1**: illustrates an example of an arrangement for measuring a biomarker value of a milk sample of an animal.
- **Figure 2A**: illustrates a cassette inserted into a milk analysis apparatus, according to an embodiment.
- **Figure 2B**: illustrates a section of a tape of the cassette comprising lateral flow sticks, according to an embodiment.
- **Figure 3A**: illustrates a housing of the milk analysis apparatus and temperature adjusting features thereof, according to an embodiment.
- **Figure 3B**: illustrates a cassette inserted into the milk analysis apparatus, according to an embodiment.
- **Figure 4**: illustrates a cassette comprising lateral flow sticks, according to an embodiment.
- **Figure 5A**: illustrates a housing of the milk analysis apparatus and a guided surface.
- **Figure 5B**: illustrates an outlet of a receiving section of the milk analysis apparatus.

### DETAILED DESCRIPTION

Embodiments of the invention described herein are defined as a milk analysis apparatus, which may be put into practice in the embodiments described below. These embodiments may, however, be exemplified and realised in many different forms and are not to be limited to the examples set forth herein; rather, these illustrative examples of embodiments are provided so that this disclosure will be thorough and complete.

Still other objects and features may become apparent from the following detailed description, considered in conjunction with the accompanying drawings. It is to be understood, however, that the drawings are designed solely for purposes of illustration and not as a definition of the limits of the herein disclosed embodiments, for which reference is to be made to the appended claims. Further, the drawings are not necessarily drawn to scale and, unless otherwise indicated, they are merely intended to conceptually illustrate the structures and procedures described herein.

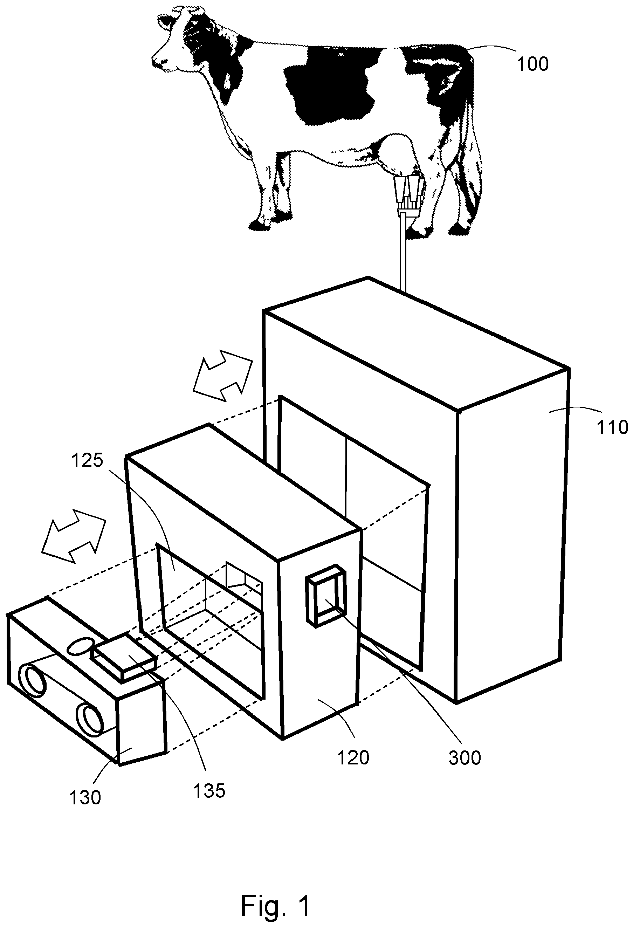

Figure 1 illustrates a scenario with an animal 100 which may be comprised in a herd of dairy animals at a dairy farm.

"Animal" may be any arbitrary type of domesticated female milk producing and/ or meat producing mammal such as cow, goat, sheep, horse, camel, dromedary, primate, dairy buffalo, donkey, reindeer, yak, etc.

Milk of the animal 100 may be extracted by a milking equipment such as e.g. a milking robot or other milking arrangement and provided to a milk analysis apparatus 120.

The milk analysis apparatus 120 may be releasably inserted into the milking equipment in some embodiments. Thus, there may be an interface between the milking equipment and the milk analysis apparatus 120 for providing milk and possibly electricity via the milking equipment to the milk analysis apparatus 120.

The milk analysis apparatus 120 comprises various electronics and equipment such as a camera, one or several pumps, a tube element for attachment to the interface to the milking equipment, motors, a communication unit etc.

A cassette 130 may be detachably inserted into, or attached to, a receiving section 125 of the milk analysis apparatus 120. The cassette 130 may comprise a tape with lateral flow sticks/ dry sticks, configured to indicate a biomarker value of a milk sample of the animal 100. The cassette 130 may in some embodiments be configured to be detachably inserted in the milk analysis apparatus 120 and held in place by a fastening means such as a snap lock, a magnet, a screw, etc., and a door of the milk analysis apparatus 120 may be closed for enclosing the cassette 130 within the milk analysis apparatus 120, thereby further fixating the cassette 130 in the position.

Thereby, a milk sample of the animal 100 may be extracted from the animal 100 by the milking equipment and provided via the milk analysis apparatus 120 to one of the lateral flow sticks on the tape of the cassette 130. The lateral flow sticks may react on presence and/ or amount of one or several biomarkers, e.g. by changing colours, or intensity of a colour. The camera in the milk analysis apparatus 120 may capture an image through an opening in the cassette 130. The captured image of the lateral flow stick may then be analysed by a control unit, and based on the intensity of the colour, presence and/ or quantity of the biomarker in the milk sample may be estimated.

The measured biomarker may be e.g. progesterone, glycoprotein, oestrogen and/ or Gonadatropin-Releasing Hormones, or any other similar biomarker associated with reproduction of the animal 100, in different embodiments.

Progesterone is a hormone that regulates several physiological functions of the animal 100. Progesterone may prepare the uterus for pregnancy, maintain the pregnancy if fertilisation occurs, and inhibit the animal 100 from showing signs of standing oestrus and ovulating when pregnant. Progesterone levels, for example, may rise at the beginning of the pregnancy, and be kept at a high level throughout the pregnancy of the animal 100. Progesterone levels in milk samples may be used to monitor pregnancy, oestrous cycles (heat detection) and/ or postpartum ovarian activity. For these reasons, progesterone levels of animals 100 at the farm is interesting for the farmer to detect and keep track of.

However, the measured biomarker may in some embodiments comprise LDH (Lactate Dehydrogenase), BHB (Beta-HydroxyButyrat), urea, and/ or somatic cell count; or other biomarker related to status of the animal 100. In some embodiments, a plurality of the above enumerated biomarkers may be measured. Alternatively, in some embodiment, the farmer may subscribe to a cassette 130 comprising a certain lateral flow sticks on the tape configured to measure a biomarker, or a set of biomarkers, as selected by the farmer; and/ or different cassettes 130 comprising lateral flow sticks on the tape configured to measure different biomarkers, or sets of biomarkers, during different periods of time of the year.

In some embodiments, a dosing module 135 may also be detachably inserted into the service module 120. The dosing module 135 may comprise for example a needle, and/ or one or several pumps. A diluent container with diluent may be external to the dosing module 135.

Thus, the milk analysis apparatus 120 comprises several modules such as the cassette 130, the dosing module 135 and/ or the liquid container, which are to be changed for a new respective module at particular time intervals when renewal or replacement is required, which may occur at different moments in time for the different modules; or alternatively synchronised with each other.

The various modules, i.e. the dosing module 135, the liquid container and/ or the cassette 130 may comprise fastening means, e.g. in form of a snap fit arrangement, magnetics, screw joints, etc., arranged to attach the respective module onto the milk analysis apparatus 120.

The modular structure of the provided solution has several advantages. By keeping the arrangement modular in form of the dosing module 135, the liquid container and the cassette 130; costs, maintenance and work intensity of the farmer may be minimised or at least reduced. Also, by separating the consumable material such as milk analysis units/ measurement sticks of the cassette 130, from elements subject to wear, like the pumps of the dosing module 135, and the electronics and instruments of the milk analysis apparatus; the liquid container could be continuously replaced with another replacement liquid container e.g. ordered via a courier service or postal office subscription.

However, the lateral flow sticks of the cassette 130 and possibly also other equipment or parts of the dosing module 135, the liquid container and/ or the milk analysis apparatus 120 may be affected when exposed to a temperature exceeding extreme threshold limits such as e.g. exceeding 30 degrees or being lower than 20 degrees, leading to incorrect test results of the analysis (or an extended incubation time of the test when the temperature is too low).

The temperature inside the milk analysis apparatus 120 may be increased due to a number of unfortunate factors, e.g. if they are placed close to a heat emitting device, exposed to inflowing sunshine, etc., which may be difficult to predict and/ or avoid.

The milk analysis apparatus 120 according to the current solution is configured for determining and adjusting the temperature inside the milk analysis apparatus 120 and thereby also temperature of the lateral flow sticks of the cassette 130, in particular the unused lateral flow sticks. Hereby, the result of the biomarker test becomes trustworthy and reliable by maintaining the temperature within a temperature threshold interval. The temperature threshold interval may be set to for example between about 20-30 degrees, etc. The temperature threshold interval may be predetermined or configurable in different embodiments.

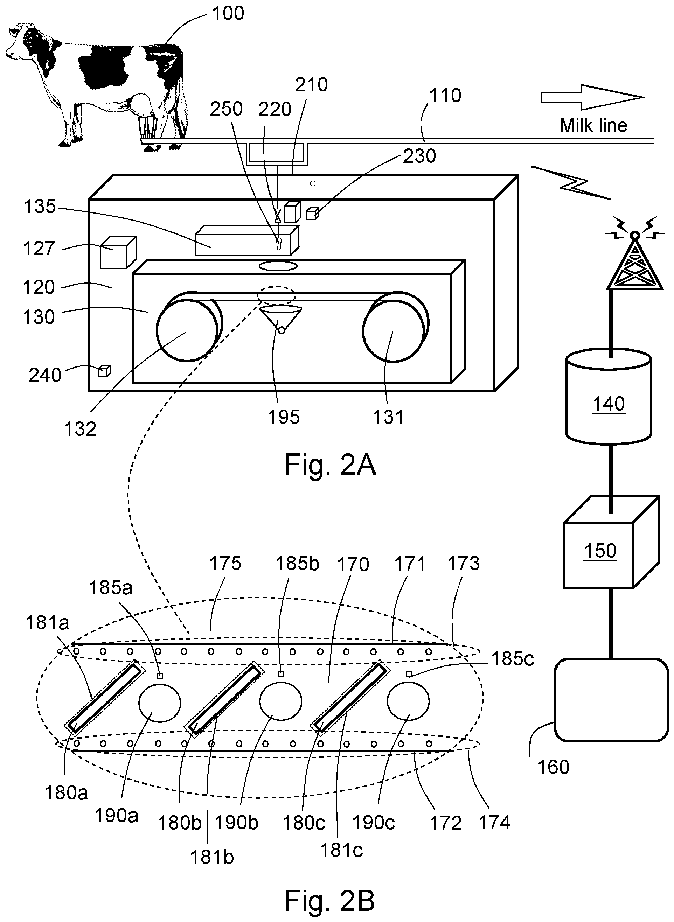

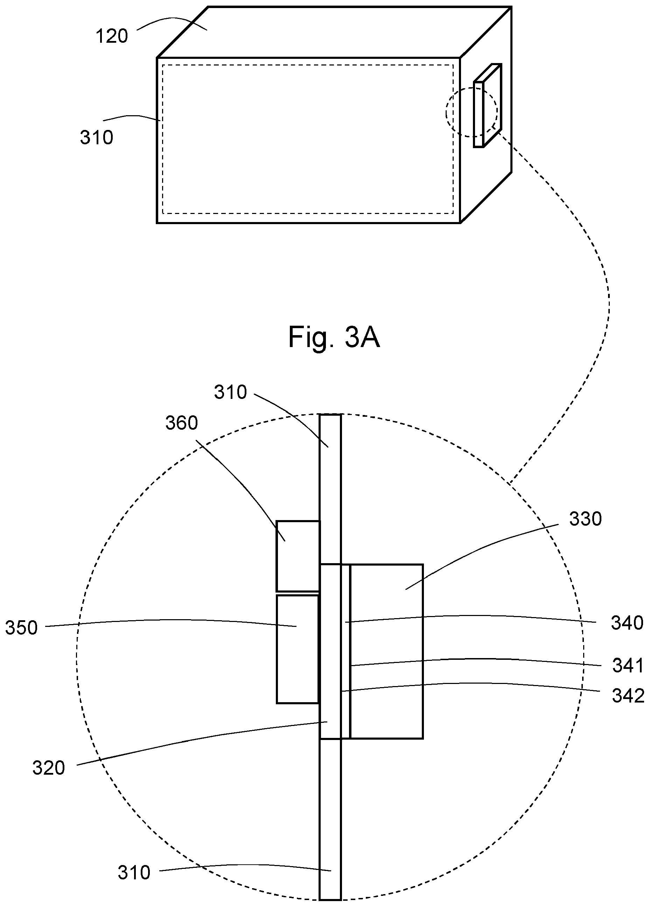

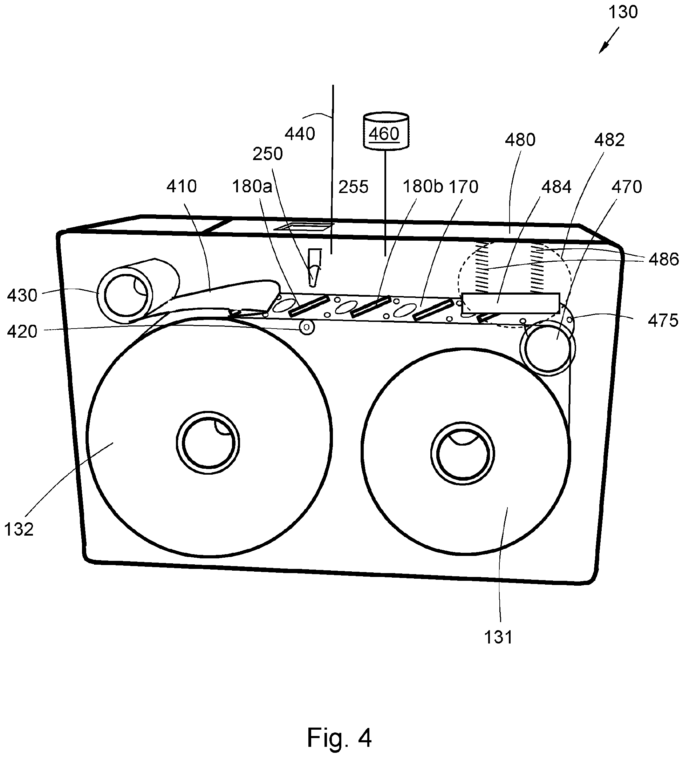

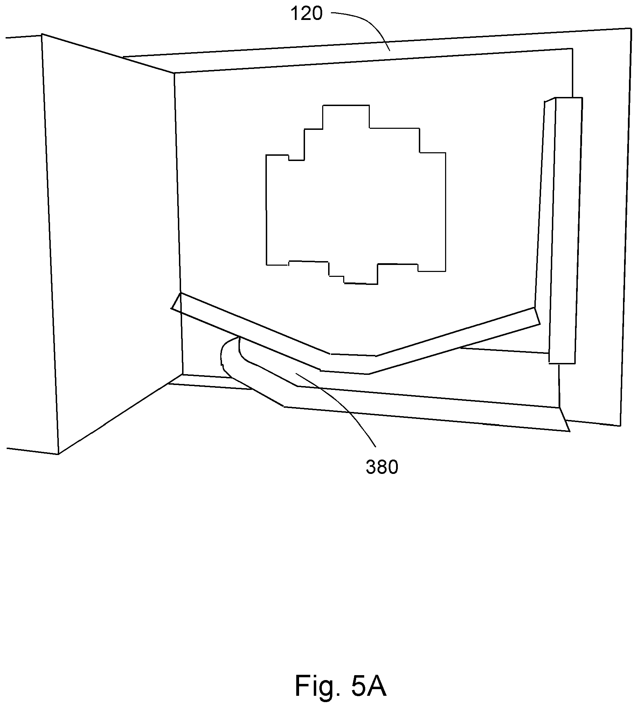

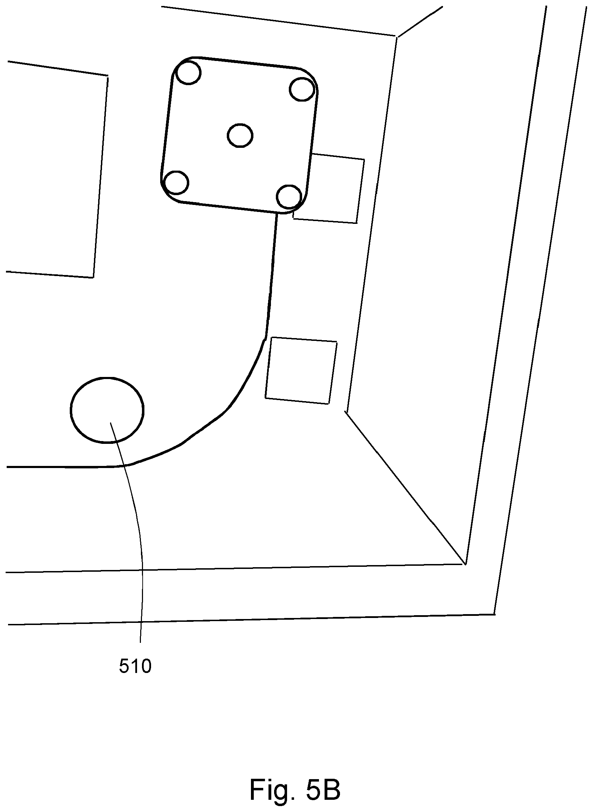

Figure 1 and Figure 2A depict general overviews of the environment in which the tape according to the provided solution is intended to operate, without going too much into details, in order for the reader to get a rough overview. Examples of details of the involved entities, in particular the cassette 130 and the tape, and how they interact with each other is disclosed in Figure 2B. Figures 3A-3B disclose the milk analysis apparatus 120 while Figure 4 illustrates a cassette 130. Figures 5A and 5B illustrates the cooperation and interaction between a guided surface of the milk analysis apparatus 120 and an outlet of the receiving section of the milk analysis apparatus 120.

Figure 2A illustrates a scenario illustrating a milk analysis apparatus 120, a cassette 130, and a dosing module 135, according to an embodiment. The milk analysis apparatus 120 comprises electronics and equipment such as e.g. a camera 210, a tube element 220 for attachment to the milking equipment, a motor, a communication unit 230, etc., to be used for determining a biometric value of a milk sample received from an animal 100. In some embodiments, the dosing module 135 may comprise one or several pumps configured to act on the tube element 220 for advancing the milk sample through the tube element 220.

In the illustrated embodiment, the dosing module 135 may comprise a needle 250 for applying the milk sample to a lateral flow stick 180a, 180b, 180c on a tape 170 in the cassette 130 through an opening in the cassette 130. The camera 210 may then align the needle 250 with the next unused lateral flow stick 180a, 180b, 180c on the tape 170 of the cassette 130.

The cassette 130 may comprise a first part 131 for maintaining unused lateral flow sticks 180a, 180b, 180c and a second part 132 for maintaining used lateral flow sticks 180a, 180b, 180c.

The camera 210 of the milk analysis apparatus 120 may capture an image of the lateral flow sticks 180a, 180b, 180c of the carrier tape 170 through the opening, and based on these images, a cassette external motor may adjust the tape 170 for positioning a new lateral flow stick 180a, 180b, 180c, on which a new test is to be made, in relation to the needle 250.

The communication unit 230 may communicate via a wired or wireless communication interface, with a control unit 150, a database 140, and/ or an output unit 160.

Such wireless communication interface may comprise, or at least be inspired by wireless communication technology such as Wi-Fi, 3GPP LTE, Bluetooth (BT) to name but a few possible examples of wireless communications in some embodiments.

The camera 210 of the milk analysis apparatus 120 is configured to inspect one lateral flow stick 180a, 180b, 180c on the tape 170 of the cassette 130, through the opening of the cassette 130. The camera 210 may also assist in alignment of the needle 250 and the position of the lateral flow stick 180a, 180b, 180c on the tape 170, by adjusting the tape 170.

Further, the milk analysis apparatus 120 also comprises a tube element 220 configured to receive the milk sample of the animal 100 via a milking equipment and provide the milk sample to a needle 250, i.e. the needle 250 comprised in the dosing module 135.

The dosing module 135 may in addition comprise at least one pump in some embodiments, configured to act on the tube element 220 for providing the milk sample to the needle 250. The pump may thus act on the tube element 220 to get the milk sample to propagate through the tube element 220, to reach the needle 250; or the mixing chamber 255 of the needle 250. The mixing chamber 255 may alternatively be external to the needle 250.

The dosing module 135 may also comprise a liquid evacuator or drainage 195, which may collect liquid that has been output by the needle 250. The liquid, when comprising merely milk, may be returned back to the milk line in some embodiments. In other embodiments, when the milk has been mixed with diluent, the liquid may be conveyed away from the cassette 130 in order not to soak or contaminate other, unused, lateral flow sticks 180a, 180b, 180c of the tape 170 on the cassette 130.

The control unit 150 is configured to monitor and adjust temperature inside the housing 310 of the milk analysis apparatus 120 as will be discussed. Also, the control unit 150 may be configured to determine a biomarker value of the milk sample of the animal 100, based on an analysis of the image, captured by the camera 210. The control unit 150 may be comprised in the milk analysis apparatus 120 in some embodiments; or be external to the milk analysis apparatus 120.

The database 140 may store measured biometric values of the animal 100, associated with an identity reference of the animal 100 and/ or a time stamp of the measurement. Other measurements and/ or data related to the animal 100 may also be stored in the database 140, such as milk yield, e.g. measured by the milk flow meter, activity, breed, parity, rumination, lactation, resting, feed intake, energy balance, Days In Milk, milk production, age and possibly other similar animal status related parameters.

The output unit 160 may be e.g. a cellular mobile telephone, a stationary or portable computing device, a computer tablet, a display, a pair of intelligent glasses, a smart contact lens, an augmented reality device, a smart watch or similar device having a user interface and wireless communication ability.

Via the output unit 160, a farmer may take part of the result of the biomarker measurement of the milk sample. The farmer is thereby able to analyse the status of the animal 100, such as e.g., check whether the animal 100 is in heat, in case progesterone is measured.

In some embodiments, information may be sent to the farmer concerning the temperature in the milk analysis apparatus 120, and perhaps send an alert when an excessive temperature is reached in the milk analysis apparatus 120, for example due to a failure of the control unit 150 to maintain the temperature within an allowed temperature interval, such as 20-30 degrees. The failure may be a consequence of a hardware error in any of the discussed components, or an electric cut for example.

When a deviation, exceeding a first threshold limit, is detected between the outcomes of the biomarker measurement and the corresponding reference value, or the temperature limit is exceeded, an alert may be outputted to the farmer. The alert may comprise e.g. visual information, an audio message, a tactile signal or a combination thereof, encouraging the operator to further investigate the reasons for the detected deviation in result. In case a plurality of people is working with the herd, a broadcast may be made to the plurality of farmers and their respective associated output units 160, in some embodiments.

Figure 2B illustrates a tape 170 according to an embodiment. The cassette 130, which may be releasably inserted into the milk analysis apparatus 120, comprises the tape 170, which in turn comprises a plurality of lateral flow sticks 180a, 180b, 180c.

In some embodiments, the tape 170 may comprise e.g. 300-700 lateral flow sticks 180a, 180b, 180c, or preferably about 400-600 lateral flow sticks 180a, 180b, 180c. Thereby, the tape 170 of the cassette 130 may comprise enough lateral flow sticks 180a, 180b, 180c for testing milk samples of an average automatic milking robot for about a month.

The lateral flow sticks 180a, 180b, 180c may be arranged with an inclination in relation to an axis, orthogonal to a longitudinal axis of the tape 170. The inclination may for example be 15 degrees or there about, or e.g. 10-30 degrees in some embodiments (non-limiting example).

An opening 190a, 190b, 190c, may be arranged between at least some of the lateral flow sticks 180a, 180b, 180c, on the tape 170, or on a bottom film of the tape 170, i.e. between the welded seams of at least some of the lateral flow sticks 180a, 180b, 180c on the bottom film. The opening 190a, 190b, 190c is configured to convey liquid away from the lateral flow sticks 180a, 180b, 180c during cleaning, or before applying the milk sample to the lateral flow stick 180a, 180b, 180c.

Milk of a first animal 100 may contaminate a milk sample of another, subsequently tested animal. To avoid contamination, or carry over, the tubings and the needle 250 may be flushed with milk of the animal 100 to be tested before the milk sample is applied to the lateral flow stick 180a, 180b, 180c. For avoiding that the flushed milk of the animal 100 to be tested soaks and/ or contaminate other unused lateral flow sticks 180a, 180b, 180c, the flushing may be made through the opening 190a, 190b, 190c of the tape 170, e.g. by lowering the needle 250 through the opening 190a, 190b, 190c, and capture the flushed milk with a liquid evacuator 195. The liquid evacuator 195 may then via a tube convey liquid away from the cassette 130.

The tape 170, or the bottom film of the tape 170 may further comprise a reference mark! sign 185a, 185b, 185c, configured to assist a camera 210 in finding the lateral flow stick 180a, 180b, 180c. The reference mark 185a, 185b, 185c may comprise e.g. a hole, a colour mark, a barcode, a simple geometry, or similar.

The reference mark 185a, 185b, 185c may also assist the camera 210 in determining the advancement of the top film reel, to peel off the top film of the lateral flow stick 180a, 180b, 180c, enough to enable application of the milk sample to the lateral flow stick 180a, 180b, 180c, while not peeling off the top film of the next lateral flow stick 180a, 180b, 180c.

Further, the tape 170, or the bottom film of the tape 170 may comprise a first group 173 of advancement apertures 175, arranged at a first edge 171 of the tape 170; and a second group 174 of advancement apertures 175, arranged at a second edge 172 of the tape 170, or the bottom film of the tape 170.

Each lateral flow stick 180a, 180b, 180c may be separately arranged on the tape 170, or the bottom film of the tape 170, by a welded seam, and wherein the sealed lateral flow sticks 180a, 180b, 180c are arranged on a distance from each other.

Figure 3A illustrates a milk analysis apparatus 120 associated with a milking equipment 110 and various temperature adjusting/ maintaining features thereof, according to an embodiment.

The milk analysis apparatus 120 comprises a housing 310 that encloses at least a part of the milk analysis apparatus 120. The housing 310 may be insulated in some embodiments.

The insulation may be made e.g. by arranging two walls at a distance from each other, wherein the distance comprises air, evacuated air ("vacuum"), polyurethane, fiberglass, polystyrene, polyethylene foam, Styrofoam or similar material.

An advantage with insulating the housing 310 is that it becomes easier to maintain the temperature in the housing 310 within a temperature interval, for example between 20-30 degrees.

The cassette 130 and thereby also the lateral flow sticks 180a, 180b, 180c maintained therein and sensible electronics of the milk analysis apparatus 120 may be maintained inside the temperature adjusted housing 310.

The milk analysis apparatus 120 may also comprise an exterior side heat exchange element 330 arranged on the external side of the housing 310 of the milk analysis apparatus 120, in some embodiments.

Furthermore, the milk analysis apparatus 120 comprises a thermoelectric element 340. The thermoelectric element 340 may comprise a Peltier element, a solid-state refrigerator, a thermoelectric cooler or similar device. The thermoelectric element 340 utilise a thermoelectric effect for converting electric voltage to a temperature difference between a first side 341 and a second side 342 of the thermoelectric element 340 via a thermocouple. When voltage is applied to the thermoelectric element 340 heat is transferred from one side 341 to the other 342, creating a temperature difference between these two sides 341, 342. At the atomic scale, an applied temperature gradient causes charge carriers in the material to diffuse from the first side 341 to the second side 342. Thereby, the thermoelectric element 340 becomes with a hot side and a cold side. The temperature difference between the first side 341 and the second side 342 of the thermoelectric element 340 is proportional to the applied voltage. Further, the hot side and the cold side of the thermoelectric element 340 may be reversed by reversing the voltage applied to the thermoelectric element 340, i.e. the direction of heating and cooling is determined by the polarity of the applied voltage. The thermoelectric element 340 may thereby be utilised as a temperature controller of the milk analysis apparatus 120.

The thermoelectric element 340 may be arranged on the external side of the housing 310 of the milk analysis apparatus 120 with the first side 341 adjacent to the exterior heat exchange element 330 and the second side 342 adjacent to the housing 310.

The milk analysis apparatus 120 may also comprise an interior heat exchange element 350 which may be arranged on the internal side of the housing 310 of the milk analysis apparatus 120, adjacent to the second side 342 of the thermoelectric element 340. Further, the milk analysis apparatus 120 may comprise a fan 360, arranged for circulating air inside the housing 310 of the milk analysis apparatus 120.

In the illustrated embodiment, the fan 360 may be arranged internal to the housing 310, for creating an airflow through the interior heat exchange element 350, which may be either warm or cold dependent on whether heating or cooling is desired.

The fan 360 is a radial fan, arranged on the interior side of the housing 310, for blowing air through the interior heat exchange element 350. In other embodiments, not being part of the present invention, the fan 360 may comprise a crossflow fan, a tangential fan, a centrifugal fan, an axial flow fan, etc.

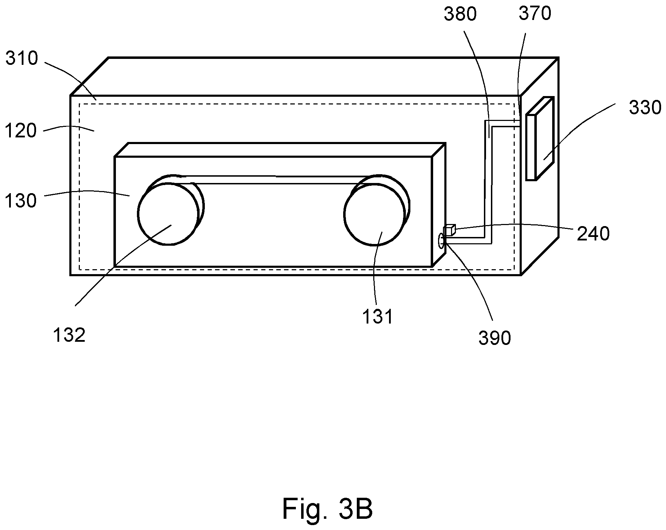

Furthermore, the milk analysis apparatus 120 may comprise a guided surface 380 as schematically illustrated in Figure 3B, for bringing air inside the housing 310 to circulate, passing the interior heat exchange element 350, when acted upon by the fan 360. The guided surface 380 may be part of an internal wall of the milk analysis apparatus 120 in some embodiments. The interior of the housing 310 is a closed compartment.

The guided surface 380 may comprise plastic channels or walls for guiding the created airflow inside the milk analysis apparatus 120, in particular for directing the airflow towards the most temperature sensible parts or elements of the milk analysis apparatus 120; and in particular the first part 131 of the cassette 130 wherein the unused lateral flow sticks 180a, 180b, 180c are maintained.

In some embodiments wherein the milk analysis apparatus 120 comprises lateral flow sticks 180a, 180b, 180c, the guided surface 380 may be arranged to lead air from the interior heat exchange element 350 to the lateral flow sticks 180a, 180b, 180c.

The guided surface 380 may comprise a first segment for guiding circulating air inside the housing 310 to pass the interior heat exchange element 350 towards an outlet of the receiving section 125 of the milk analysis apparatus 120. Also, the guided surface 380 may comprise a second segment for guiding circulating air inside the housing 310 from the outlet of the receiving section 125 back towards the interior heat exchange element 350.

The lateral flow sticks 180a, 180b, 180c may thus be comprised in the cassette 130 which is detachably inserted into the receiving section 125 of the milk analysis apparatus 120. The cassette 130 may comprise an air guidance means 390 for directing temperature adjusted air provided by the guided surface 380 to the unused lateral flow sticks 180a, 180b, 180c in a first section 131 of the cassette 130, for example on a tape 170, via the outlet of the receiving section 125 of the milk analysis apparatus 120 as illustrated in Figure 3B.

The milk analysis apparatus 120 furthermore comprises a temperature sensor 240, i.e. a thermometer, arranged inside the housing 310. The temperature sensor 240 may be arranged adjacent to the outlet of the receiving section 125 of the milk analysis apparatus 120 and thereby also adjacent to the air guidance means 390 of the cassette 130 in some embodiments.

By making the temperature measurements close to the location of the unused lateral flow sticks 180a, 180b, 180c in the first section 131 of the cassette 130, the temperature adjustments may be made based on an estimated temperature of the unused lateral flow sticks 180a, 180b, 180c.

By continuously or at certain time intervals measure the temperature inside the housing 310, this temperature may be compared with a predetermined temperature threshold limit and the temperature then adjusted to maintain the temperature below the threshold limit; or within a predetermined temperature interval such as about 20-30 degrees.

The milk analysis apparatus 120 in addition may comprise or be connected to a control unit 150. The control unit 150 may be in the milk analysis apparatus 120, or possibly be situated external to the same. The control unit 150 may be configured to determine temperature inside the housing 310 via the temperature sensor 240. Also, the control unit 150 may furthermore be configured to compare the determined temperature with a temperature threshold limit, or with an upper temperature threshold limit set to about 30 degrees and/ or a lower temperature threshold limit set to about 20 degrees.

Based on the outcome of the comparison, the control unit 150 then may adjust current provided to the thermoelectric element 340. When the second side 342 of the thermoelectric element 340 is the hot side, the first side 341 of the thermoelectric element 340 is the cold side, and vice versa. The hot side and the cold side of the thermoelectric element 340 could be reversed by reversing the current provided to the thermoelectric element 340. The control unit 150 could hereby adjust whether heating or cooling of the inside of the housing 310 is desired.

The extent of the heating/ cooling respectively of the respective sides 341, 342 of the thermoelectric element 340 may be increased by increasing the current provided to the thermoelectric element 340 (and vice versa).

The control unit 150 may comprise a processing circuitry comprising one or more instances of a processing circuit, i.e. a Central Processing Unit (CPU), a processor, a processing unit, an Application Specific Integrated Circuit (ASIC), a microprocessor, a Graphics Processing Unit (GPU), or other processing logic that may interpret and execute instructions.

Communication between the control unit 150 and the temperature sensor 240 may be made over a wired or wireless communication interface.

In some embodiments, the milk analysis apparatus 120 may comprise a thermal conducting plate 320 arranged in an opening of the housing 310, for transferring heat in to/ out of the milk analysis apparatus 120. The second side 342 of the thermoelectric element 340 may be situated adjacent to the exterior side of the thermal conducting plate 320 while and the interior heat exchange element 350 may be situated adjacent to the interior side of the thermal conducting plate 320.

The thermal conducting plate 320 may with advantage be made of a material having good thermal conducting properties such as copper, aluminium, boron arsenide or similar material, or an alloy comprising any of these materials, or any other material with similar properties.

Aluminium is a metal having excellent thermal conducting properties, while being relatively cheap, lightweight and is easily machined. The low melting temperature of 660°C allows for easy casting. By arranging the thermal conducting plate 320 in the opening of the housing 310, heat/ cold absorbed from the interior heat exchange element 350 inside the milk analysis apparatus 120 may be transferred to the second side 342 of the thermoelectric element 340. The temperature inside the milk analysis apparatus 120 is thereby controlled while maintaining the closed design of the housing 310 of the milk analysis apparatus 120.

By having a closed design of the housing 310 and the milk analysis apparatus 120, it is avoided that dirt, moisture etc., i.e. particles which may affect the performance of the electronics and/ or the lateral flow sticks 180a, 180b, 180c, is allowed to enter the housing 310. Thanks to the thermal conducting plate 320, heat cold be transferred into, or be evacuated from the housing 310 of the milk analysis apparatus 120 also when the housing 310 creates a closed compartment. Thereby temperature may be adjusted within the housing 310 without allowing dust, dirt, insects or similar to enter the housing 310 of the milk analysis apparatus 120.

The thermoelectric element 340 may be arranged on one of the external lateral sides of the milk analysis apparatus 120. An advantage therewith is that moisture damage of the thermoelectric element 340, which is sensitive for humidity, is avoided. At least the risk or probability for moisture damage is radically reduced. The thermoelectric element 340 such as Peltier elements are very sensible for liquid. Hereby, reliability of the milk analysis apparatus 120 is increased.

Thereby, temperature within the milk analysis apparatus 120 and/ or the cassette 130 may be kept at the same level between about e.g. 20-30 degrees. The predetermined temperature threshold limit may be set to e.g. 25 degrees, 28 degrees, 24-30 degrees Celsius, etc. In case the temperature as measured by the temperature sensor 240 exceeds the temperature threshold limit, the control unit 150 may be activated for cooling the temperature. Further, any entered humidity within the cassette 130 or the milk analysis apparatus 120 may condensate on the interior heat exchange element 350 inside the milk analysis apparatus 120, drip off and leave the housing 310 of the milk analysis apparatus 120 via a drain at the bottom.

Figure 4 illustrates details of the cassette 130, which may be releasably inserted into the receiving section 125 of the milk analysis apparatus 120.

The cassette 130 may comprise a first part 131 for maintaining unused lateral flow sticks 180a, 180b, 180c and a second part 132 for maintaining used lateral flow sticks 180a, 180b, 180c. Also, the cassette 130 may comprise an air guidance means 390 arranged on the first part 131 of the cassette 130 wherein the unused lateral flow sticks 180a, 180b, 180c are maintained. The lateral flow sticks 180a, 180b, 180c may be separately, i.e. individually, arranged on the tape 170 at a distance from each other.

In the illustrated embodiment, the tape 170 comprises a bottom film, comprising the lateral flow sticks 180a, 180b, 180c, covered with a top film 410 configured to seal the lateral flow sticks 180a, 180b, 180c on the tape 170.

Also, in the illustrated embodiment, a top film reel 430 is comprised, arranged to peel off and collect the top film 410 of the tape 170. The top film 410 may be peeled off just before the milk sample is to be applied to the peeled off lateral flow sticks 180a, 180b, 180c.

Further, the cassette 130 may comprise a capstan reel 470 comprising teeth 475, for engaging with advancement apertures 175 on the tape 170.

The cassette 130 may in addition also comprise a top lid 480, comprising the opening 135, configured to enable the needle 250 of the milk analysis apparatus 120 to be inserted for applying the milk sample of the animal 100 to one of the lateral flow sticks 180a, 180b, 180c, to which the top film 410 has been peeled off.

The top lid 480 may comprise at least one pressure exertion member 482, arranged to act on the tape 170 to keep it at a predetermined distance from the top lid 480. Thereby, the camera 210 may be focused on the lateral flow sticks 180a, 180b, 180c, as they are constantly situated at the same distance from the camera 210. The pressure exertion member 482 may comprise a spring 486, or a flexible material, and a tape interface unit 484.

Also, the top lid 480 may act on the tape 170 to keep the advancement apertures 175 on the tape 170 in place at the teeth 475 on the capstan reel 470. The form of the top lid 480 may thus have a shape keeping the advancement apertures 175 on the tape 170 in place at the teeth 475 on the capstan reel 470.

Some embodiments of the cassette 130 may comprise a tape supporting member 320, arranged to guide the tape 170 in a trajectory between the tape distributing spool, and the capstan reel 470. The tape supporting member 420 may ascertain that the tape 170 is kept at the predetermined distance from the camera 210, thereby enabling the camera 210 to be focused on the lateral flow sticks 180a, 180b, 180c.

In some embodiments, the tape 170 may comprise e.g. 300-700 lateral flow sticks 180a, 180b, 180c, or preferably about 400-600 lateral flow sticks 180a, 180b, 180c. Thereby, the tape 170 of the cassette 130 may comprise enough lateral flow sticks 180a, 180b, 180c for testing milk samples of an average automatic milking robot for about a month. The cassette 130 with the tape 170 and the lateral flow sticks 180a, 180b, 180c may then be wasted and exchanged for another one, e.g. through a service subscription. In some embodiments, the cassette 130 may be recycled; i.e. the used cassette 130 may be opened and the used tape 170 may be removed from the used cassette 130. Then, a new, unutilised tape 170 with lateral flow sticks 180a, 180b, 180c may be inserted into the cassette 130, and the cassette 130 may be reassembled.

The lateral flow sticks 180a, 180b, 180c may be designed for one-time usage each. In some embodiments, the lateral flow sticks 180a, 180b, 180c of the tape 170 may be configured to change colour or colour nuance when exposed to the biomarker.

The camera 210 in the milk analysis apparatus 120 may capture an image of the lateral flow sticks 180a, 180b, 180c in question, after a predetermined or configurable time period. The colour, or colour intensity of the lateral flow sticks 180a, 180b, 180c on the captured image may then be analysed by the control unit 150, where different colour intensities may be associated with a certain biomarker level of the milk sample.

The milk sample may in some embodiments be mixed with a diluent before being applied to the lateral flow sticks 180a, 180b, 180c on the tape 170, e.g. in the mixing chamber 255.

In some embodiments, the cassette 130 may be sealed from the environment and thereby create a climate chamber, wherein a climate environment prevails in the cassette 130. The cassette 130 thereby becomes isolated from environmental impact of dust, dirt, liquids, etc., of the farm.

When the tape 170 is moved for placing the lateral flow sticks 180a, 180b, 180c to be used in position aligned with the needle 250, the milk/ diluent mix may be applied on the lateral flow sticks 180a, 180b, 180c.

An advantage of the disclosed solution, by making a division between the milk analysis apparatus 120 comprising camera, motor, pumps and other electronics and/ or apparatuses; and one or several cassettes or modules 130, 460 comprising disposable material, the solution becomes very easy to use for the operator.

The cassette 130 may comprise lateral flow sticks 180a, 180b, 180c etc., for supporting the farm for a certain predetermined period of time, such as e.g. a month, two months, etc. Before the end of that time period, a supplier may provide a new cassette 130 to the farm, which the operator easy may put into the milk analysis apparatus 120, without having to interact with the sensible electronics of the milk analysis apparatus 120. The used cassette 130 may then be disposed.

Figure 5A illustrates a guided surface 380 which optionally may be part of an internal wall of the milk analysis apparatus 120, for example created by injection moulding or similar technology. The guided surface 380 may comprise a first segment for guiding circulating air inside the housing 310 to pass the interior heat exchange element 350 towards the outlet 510 of the receiving section 125, which is illustrated in Figure 5B.

The guided surface 380 may also comprise a second segment for guiding circulating air inside the housing 310 from the outlet 510 of the receiving section 125 back towards the interior heat exchange element 350.

When the cassette 130 as illustrated in Figures 2A, 3B and 4 is releasably inserted into the receiving section 125 of the milk analysis apparatus 120, the air guidance means 390 arranged on the first part 131 of the cassette 130 becomes situated adjacent to the outlet 510 of the receiving section 125.

Air guided through the guided surface 380 to the outlet 510 of the receiving section 125 may thereby be further guided via the air guidance means 390 towards the first part 131 of the cassette 130 wherein the unused lateral flow sticks 180a, 180b, 180c are maintained, which thereby may be kept within the predetermined temperature interval, 20-30 degrees.

The embodiments, or parts thereof, illustrated in any one of Figure 1, Figure 2A, Figure 2B, Figure 3A, Figure 3B, Figure 4, Figure 5A and/ or Figure 5B may with advantage be combined with each other for achieving further benefits.

The terminology used in the description of the embodiments as illustrated in the accompanying drawings is not intended to be limiting of the described cassette 130, milk analysis apparatus 120, and/ or control unit 150. Various changes, substitutions and/ or alterations may be made, without departing from invention embodiments as defined by the appended claims.

As used herein, the term "and/ or" comprises any and all combinations of one or more of the associated listed items. The term "or" as used herein, is to be interpreted as a mathematical OR, i.e., as an inclusive disjunction; not as a mathematical exclusive OR (XOR), unless expressly stated otherwise. In addition, the singular forms "a", "an" and "the" are to be interpreted as "at least one", thus also possibly comprising a plurality of entities of the same kind, unless expressly stated otherwise. It will be further understood that the terms "includes", "comprises", "including" and/ or "comprising", specifies the presence of stated features, actions, integers, steps, operations, elements, and/ or components, but do not preclude the presence or addition of one or more other features, actions, integers, steps, operations, elements, components, and/ or groups thereof. A single unit such as e.g. a processor may fulfil the functions of several items recited in the claims. The mere fact that certain measures or features are recited in mutually different dependent claims, illustrated in different figures or discussed in conjunction with different embodiments does not indicate that a combination of these measures or features cannot be used to advantage. A computer program may be stored/ distributed on a suitable medium, such as an optical storage medium or a solid-state medium supplied together with or as part of other hardware but may also be distributed in other forms such as via Internet or other wired or wireless communication system.

## Claims

1. A milk analysis apparatus (120) associated with a milking equipment (110), configured for receiving a cassette (130) detachably inserted into the milk analysis apparatus (120); wherein the milk analysis apparatus (120) comprises:
a receiving section (125), configured to detachably receive the cassette (130);
a housing (310) enclosing at least a part of the milk analysis apparatus (120);
a thermoelectric element (340);
an interior heat exchange element (350), arranged adjacent to the thermoelectric element (340);
a fan (360), arranged for circulating air inside the housing (310) of the milk analysis apparatus (120);
a temperature sensor (240) arranged inside the housing (310) of the milk analysis apparatus (120);
a guided surface (380) of the milk analysis apparatus (120) for guiding circulating air inside the housing (310) via the interior heat exchange element (350) towards an outlet (510) of the receiving section (125); and
a control unit (150) configured to:
determine temperature inside the housing (310) via the temperature sensor (230);
compare the determined temperature with a temperature threshold limit; and
adjust current provided to the thermoelectric element (340), based on the comparison, wherein the fan (360) is a radial fan, arranged on the interior of the housing (310), for creating an airflow through the interior heat exchange element (350), thereby bringing the air to circulate within the housing (310).

2. The milk analysis apparatus (120) according to claim 1, wherein the milk analysis apparatus (120) comprises:
an exterior heat exchange element (330) arranged on the external side of the housing (310);
the interior heat exchange element (350) arranged on the internal side of the housing (310) of the milk analysis apparatus (120), adjacent to the thermoelectric element (340); and
wherein the thermoelectric element (340) is arranged on the external side of the housing (310) of the milk analysis apparatus (120) with a first side (341) adjacent to the exterior heat exchange element (330) and a second side (342) adjacent to the housing (310).

3. The milk analysis apparatus (120) according to claim 2, comprising:
a thermal conducting plate (320) arranged in an opening of the housing (310); and wherein the second side (342) of the thermoelectric element (340) is situated adjacent to the exterior side of the thermal conducting plate (320); and the interior heat exchange element (350) is situated adjacent to the interior side of the thermal conducting plate (320).

4. The milk analysis apparatus (120) according to any one of claims 1-3, wherein the guided surface (380) comprises:
a first segment for guiding circulating air inside the housing (310) to pass the interior heat exchange element (350) towards the outlet (510) of the receiving section (125); and
a second segment for guiding circulating air inside the housing (310) from the outlet (510) of the receiving section (125) back towards the interior heat exchange element (350).

5. The milk analysis apparatus (120) according to any one of claims 1-4, wherein the temperature sensor (230) is arranged adjacent to the outlet (510) of the receiving section (125).

6. The milk analysis apparatus (120) according to any one of claims 1-5, wherein the thermoelectric element (340) is arranged on one of the external lateral sides of the milk analysis apparatus (120).

7. The milk analysis apparatus (120) according to any one of claims 1-6, wherein the housing (310) enclosing the milk analysis apparatus (120) comprises an insulated housing (310).

8. The milk analysis apparatus (120) according to any one of claims 1-7, wherein the guided surface (380) is part of an internal wall of the milk analysis apparatus (120).

9. The milk analysis apparatus (120) according to any one of claims 1-8, wherein the control unit (150) configured to:
compare the determined temperature with an upper temperature threshold limit set to about 30 degrees and a lower temperature threshold limit set to about 20 degrees; and
increase current provided to the thermoelectric element (340) for increasing the temperature when the temperature inside the housing (310) is determined to be lower than the lower temperature threshold limit; or
decrease current provided to the thermoelectric element (340) for decreasing the temperature when the temperature inside the housing (310) is determined to be higher than the upper temperature threshold limit.

10. The milk analysis apparatus (120) according to any one of claims 1-9, wherein the control unit (150) is configured to:
adjust direction of heating and/ or cooling of the thermoelectric element (340) by adjusting polarity of the applied voltage to the thermoelectric element (340).

11. The milk analysis apparatus (120) according to claim 10, wherein the control unit (150) is configured to:
increase temperature output of the thermoelectric element (340) by increasing current provided to the thermoelectric element (340).

## Patentansprüche

1. Milchanalysevorrichtung (120), die einer Melkanlage (110) zugeordnet ist, die zum Aufnehmen einer Kassette (130) konfiguriert ist, die in die Milchanalysevorrichtung (120) lösbar eingeführt ist;
wobei die Milchanalysevorrichtung (120) umfasst:
einen Aufnahmeabschnitt (125), der konfiguriert ist, um die Kassette (130) lösbar aufzunehmen;
ein Gehäuse (310), das mindestens einen Teil der Milchanalysevorrichtung (120) umschließt;
ein thermoelektrisches Element (340);
ein Innenwärmetauschelement (350), das angrenzend an das thermoelektrische Element (340) angeordnet ist;
einen Lüfter (360), der zum Zirkulieren von Luft innerhalb des Gehäuses (310) der Milchanalysevorrichtung (120) angeordnet ist;
einen Temperatursensor (240), der innerhalb des Gehäuses (310) der Milchanalysevorrichtung (120) angeordnet ist;
eine geführte Oberfläche (380) der Milchanalysevorrichtung (120) zum Führen von zirkulierender Luft innerhalb des Gehäuses (310) über das Innenwärmetauschelement (350) zu einem Auslass (510) des Aufnahmeabschnitts (125) hin; und
eine Steuereinheit (150), die konfiguriert ist, zum:
Bestimmen von Temperatur innerhalb des Gehäuses (310) über den Temperatursensor (230);
Vergleichen der bestimmten Temperatur mit einer Temperaturschwellengrenze; und
Einstellen von Strom, der an das thermoelektrische Element (340) bereitgestellt wird, basierend auf dem Vergleich, wobei der Lüfter (360) ein Radiallüfter ist, der an dem Inneren des Gehäuses (310) angeordnet ist, zum Erzeugen eines Luftstroms durch das Innenwärmetauschelement (350), wodurch die Luft innerhalb des Gehäuses (310) in Zirkulation gebracht wird.

2. Milchanalysevorrichtung (120) nach Anspruch 1, wobei die Milchanalysevorrichtung (120) umfasst:
ein Außenwärmetauschelement (330), das an der Außenseite des Gehäuses (310) angeordnet ist;
das Innenwärmetauschelement (350), das an der Innenseite des Gehäuses (310) der Milchanalysevorrichtung (120) angrenzend an das thermoelektrische Element (340) angeordnet ist; und
wobei das thermoelektrische Element (340) an der Außenseite des Gehäuses (310) der Milchanalysevorrichtung (120) mit einer ersten Seite (341) angrenzend an das Außenwärmetauschelement (330) und einer zweiten Seite (342) angrenzend an das Gehäuse (310) angeordnet ist.

3. Milchanalysevorrichtung (120) nach Anspruch 2, umfassend:
eine Wärmeleitplatte (320), die in einer Öffnung des Gehäuses (310) angeordnet ist; und wobei die zweite Seite (342) des thermoelektrischen Elements (340) angrenzend an die Außenseite der Wärmeleitplatte (320) gelegen ist; und das Innenwärmetauschelement (350) angrenzend an die Innenseite der Wärmeleitplatte (320) gelegen ist.

4. Milchanalysevorrichtung (120) nach einem der Ansprüche 1 bis 3, wobei die geführte Oberfläche (380) umfasst:
ein erstes Segment zum Führen von zirkulierender Luft innerhalb des Gehäuses (310), um das Innenwärmetauschelement (350) zu dem Auslass (510) des Aufnahmeabschnitts (125) hin zu passieren; und
ein zweites Segment zum Führen von zirkulierender Luft innerhalb des Gehäuses (310) von dem Auslass (510) des Aufnahmeabschnitts (125) zurück zu dem Innenwärmetauschelement (350) hin.

5. Milchanalysevorrichtung (120) nach einem der Ansprüche 1 bis 4, wobei der Temperatursensor (230) angrenzend an den Auslass (510) des Aufnahmeabschnitts (125) angeordnet ist.

6. Milchanalysevorrichtung (120) nach einem der Ansprüche 1 bis 5, wobei das thermoelektrische Element (340) auf einer der äußeren lateralen Seiten der Milchanalysevorrichtung (120) angeordnet ist.

7. Milchanalysevorrichtung (120) nach einem der Ansprüche 1 bis 6, wobei das Gehäuse (310), das die Milchanalysevorrichtung (120) umschließt, ein isoliertes Gehäuse (310) umfasst.

8. Milchanalysevorrichtung (120) nach einem der Ansprüche 1 bis 7, wobei die geführte Oberfläche (380) Teil einer Innenwand der Milchanalysevorrichtung (120) ist.

9. Milchanalysevorrichtung (120) nach einem der Ansprüche 1 bis 8, wobei die Steuereinheit (150) konfiguriert ist zum:
Vergleichen der bestimmten Temperatur mit einer oberen Temperaturschwellengrenze, die auf etwa 30 Grad festgelegt ist, und einer unteren Temperaturschwellengrenze, die auf etwa 20 Grad festgelegt ist; und
Erhöhen des Stroms, der dem thermoelektrischen Element (340) bereitgestellt wird, zum Erhöhen der Temperatur, wenn bestimmt wird, dass die Temperatur innerhalb des Gehäuses (310) niedriger als die untere Temperaturschwellengrenze ist; oder
Verringern des Stroms, der dem thermoelektrischen Element (340) bereitgestellt wird, zum Verringern der Temperatur, wenn bestimmt wird, dass die Temperatur innerhalb des Gehäuses (310) höher als die obere Temperaturschwellengrenze ist.

10. Milchanalysevorrichtung (120) nach einem der Ansprüche 1 bis 9, wobei die Steuereinheit (150) konfiguriert ist zum:
Einstellen einer Richtung eines Erwärmens und/oder eines Abkühlens des thermoelektrischen Elements (340) durch Einstellen einer Polarität der angelegten Spannung an das thermoelektrische Element (340).

11. Milchanalysevorrichtung (120) nach Anspruch 10, wobei die Steuereinheit (150) konfiguriert ist zum:
Erhöhen einer Temperaturabgabe des thermoelektrischen Elements (340) durch Erhöhen des Stroms, der dem thermoelektrischen Element (340) bereitgestellt wird.

## Revendications

1. Appareil d'analyse de lait (120) associé à un équipement de traite (110), conçu pour recevoir une cassette (130) insérée de manière détachable dans l'appareil d'analyse de lait (120) ; dans lequel l'appareil d'analyse de lait (120) comprend :
une section de réception (125), conçue pour recevoir de manière détachable la cassette (130) ;
un boîtier (310) renfermant au moins une partie de l'appareil d'analyse de lait (120) ;
un élément thermoélectrique (340) ;
un élément d'échange de chaleur intérieur (350), agencé adjacent à l'élément thermoélectrique (340) ;
un ventilateur (360), agencé pour faire circuler de l'air à l'intérieur du boîtier (310) de l'appareil d'analyse du lait (120) ;
un capteur de température (240) agencé à l'intérieur du boîtier (310) de l'appareil d'analyse de lait (120) ;
une surface guidée (380) de l'appareil d'analyse de lait (120) pour guider de l'air en circulation à l'intérieur du logement (310) par l'intermédiaire de l'élément d'échange de chaleur intérieur (350) vers une sortie (510) de la section de réception (125) ; et
une unité de commande (150) configurée pour :
déterminer la température à l'intérieur du boîtier (310) par l'intermédiaire du capteur de température (230) ;
comparer la température déterminée à une limite seuil de température ; et
ajuster un courant fourni à l'élément thermoélectrique (340), sur la base de la comparaison, dans lequel le ventilateur (360) est un ventilateur radial, agencé sur l'intérieur du boîtier (310), pour créer un flux d'air par le biais de l'élément d'échange de chaleur intérieur (350), amenant ainsi l'air à circuler à l'intérieur du boîtier (310).

2. Appareil d'analyse de lait (120) selon la revendication 1, dans lequel l'appareil d'analyse de lait (120) comprend :
un élément d'échange de chaleur extérieur (330) agencé sur le côté externe du boîtier (310) ;
l'élément d'échange de chaleur intérieur (350) agencé sur le côté interne du boîtier (310) de l'appareil d'analyse de lait (120), adjacent à l'élément thermoélectrique (340) ; et
dans lequel l'élément thermoélectrique (340) est agencé sur le côté externe du boîtier (310) de l'appareil d'analyse de lait (120) avec un premier côté (341) adjacent à l'élément d'échange de chaleur extérieur (330) et un second côté (342) adjacent au boîtier (310).

3. Appareil d'analyse de lait (120) selon la revendication 2, comprenant :
une plaque thermoconductrice (320) agencée dans une ouverture du boîtier (310) ; et dans lequel le second côté (342) de l'élément thermoélectrique (340) est situé adjacent au côté extérieur de la plaque thermoconductrice (320) ; et l'élément d'échange de chaleur intérieur (350) est situé adjacent au côté intérieur de la plaque thermoconductrice (320).

4. Appareil d'analyse de lait (120) selon l'une quelconque des revendications 1 à 3, dans lequel la surface guidée (380) comprend :
un premier segment pour guider de l'air en circulation à l'intérieur du boîtier (310) pour faire passer l'élément d'échange de chaleur intérieur (350) vers la sortie (510) de la section de réception (125) ; et
un second segment pour guider l'air en circulation à l'intérieur du boîtier (310) depuis la sortie (510) de la section de réception (125) vers l'élément d'échange de chaleur intérieur (350).

5. Appareil d'analyse de lait (120) selon l'une quelconque des revendications 1 à 4, dans lequel le capteur de température (230) est agencé adjacent à la sortie (510) de la section de réception (125).

6. Appareil d'analyse de lait (120) selon l'une quelconque des revendications 1 à 5, dans lequel l'élément thermoélectrique (340) est agencé sur l'un des côtés latéraux externes de l'appareil d'analyse de lait (120).

7. Appareil d'analyse de lait (120) selon l'une quelconque des revendications 1 à 6, dans lequel le boîtier (310) renfermant l'appareil d'analyse de lait (120) comprend un boîtier isolé (310).

8. Appareil d'analyse de lait (120) selon l'une quelconque des revendications 1 à 7, dans lequel la surface guidée (380) fait partie d'une paroi interne de l'appareil d'analyse de lait (120).

9. Appareil d'analyse de lait (120) selon l'une quelconque des revendications 1 à 8, dans lequel l'unité de commande (150) est configurée pour :
comparer la température déterminée à une limite seuil de température supérieure définie à environ 30 degrés et une limite seuil de température inférieure définie à environ 20 degrés ; et
augmenter un courant fourni à l'élément thermoélectrique (340) pour augmenter la température lorsque la température à l'intérieur du boîtier (310) est déterminée comme étant inférieure à la limite seuil de température inférieure ; ou
diminuer un courant fourni à l'élément thermoélectrique (340) pour diminuer la température lorsque la température à l'intérieur du boîtier (310) est déterminée comme étant supérieure à la limite seuil de température supérieure.

10. Appareil d'analyse de lait (120) selon l'une quelconque des revendications 1 à 9, dans lequel l'unité de commande (150) est configurée pour :
ajuster une direction de chauffage et/ou de refroidissement de l'élément thermoélectrique (340) en ajustant une polarité de la tension appliquée à l'élément thermoélectrique (340).

11. Appareil d'analyse de lait (120) selon la revendication 10, dans lequel l'unité de commande (150) est configurée pour :
augmenter une sortie de température de l'élément thermoélectrique (340) en augmentant un courant fourni à l'élément thermoélectrique (340).
